# EUROPEAN PATENT APPLICATION

(11) **EP 2 077 114 A1**
(43) Date of publication of application: **08.07.2009**
(21) Application number: 07829230.7
(22) Date of filing: 04.10.2007
(51) Int. Cl.: A61K 31/635, A61K 9/22, A61K 31/5415, A61K 31/573, A61K 45/00, A61P 21/00, A61P 29/00, A61P 43/00, C07D 213/76

(54) **PHARMACEUTICAL PREPARATION FOR TREATING FIBROMYALGIA**

(30) Priority: 05.10.2006 JP 2006274464
(71) Applicant: St. Marianna University School of Medicine, Kawasaki-shi, Kanagawa 216-8511 (JP)
(72) Inventor: NISHIOKA, Kusuki, Tokyo 150-0012 (JP)
(74) Representative: Bassil, Nicholas Charles
(86) International application number: PCT/JP2007/069492
(87) International publication number: WO 2008/041751

(57) **Abstract**

It is difficult to develop pharmaceutical agents for treating fibromyalgia since both the pathogenetic cause and the onset mechanism remain to be clarified. An antirheumatic agent salazosulfapyridine was administered to fibromyalgia patients. Unexpectedly, salazosulfapyridine was seen to be effective in fibromyalgia patients in whom neither the rheumatoid factor nor an immune disorder was detectable, in particular to relieve pain of enthesitis. Furthermore, salazosulfapyridine was also effective to alleviate enthesitis and to relieve pain in fibromyalgia. In addition, the combined use of salazosulfapyridine with a corticosteroid or a nonsteroidal anti-inflammatory agent potentiated the therapeutic effect of salazosulfapyridine in clinical patients to whom salazosulfapyridine alone was not effective enough.

## Description

### Technical Field

The present invention relates to novel pharmaceutical uses of salazosulfapyridine, and specifically to agents for treating fibromyalgia which contain salazosulfapyridine.

### Background Art

Fibromyalgia is a disorder with systemic diffuse pain and fatigue as chief complaints, and objective symptoms of characteristic tender points. In addition to these, psychosomatic symptoms such as sleep disturbances, anxiety, depression, and restlessness are often observed in fibromyalgia. As the disease progresses further, frequent urination, irritable bowel syndrome, dysmenorrhea, sicca syndrome, and the like are often seen. Fibromyalgia most commonly develops among women in their 50s. Since it is frequently accompanied by a slight fever, malaise, and the like, such patients are often suspected of having rheumatoid arthritis or connective tissue disease when diagnosed. However, in general, test results of biochemical tests, immunoglobulin quantitations, and other tests including the blood sedimentation test, rheumatoid arthritis (RA) test, C reactive protein (CRP), and rheumatoid arthritis hemagglutination (RAHA) test are normal. Furthermore, no abnormal findings are seen in X-rays of the spine, large and small joints, etc.

Diagnosis of fibromyalgia may require experience because there is no known laboratory finding specific to it, and no recognizable changes in physical structure. In 1990, the American College of Rheumatology set forth two evaluation criteria for the classification of fibromyalgia: (1) the presence of persistent widespread pain and (2) pain in 11 or more of 18 specific tender points (Non-Patent Document 1). In Japan, fibromyalgia is currently diagnosed based on this classification criteria established in 1990 by the American College of Rheumatology.

Fibromyalgia pain is assumed to occur via a pathway different to the pathway of so-called ordinary pain. Specifically, in the pathway of ordinary pain, an inflammation or stimulation at each body part is transmitted to pain receptors and this stimulus travels upwards through the spine and enters the brain. As a result, the pain is perceived. On the other hand, it is said that in fibromyalgia, anxieties concerning pain trigger various psychiatric symptoms, which in turn serve as factors inducing new pain.

There have been many discussions on the pathogenesis of fibromyalgia. However, the major cause still remains unclear. Previous reports describe the correlation with central nervous system abnormalities, and genetic, psychological, and social factors, etc.

Reported central nervous system abnormalities in fibromyalgia are sleep disturbances, neuropeptide abnormalities, and functional abnormalities in brain activity. For example, 63% to 90% of fibromyalgia patients have symptoms of sleep disorders. Systemic pain may be causing a tendency towards insomnia, but unrefreshing sleep due to getting only a light sleep is said to be a characteristic feature of this disease. Yukioka advocated the use of the sleep brain wave test for fibromyalgia diagnosis (Non-Patent Document 2). According to Yukioka, prolonged stage 1 and shortened stages 3 and 4 of non-REM sleep, and reduced frequency of REM sleep are observed in fibromyalgia. In particular, focusing attention on α wave intrusion in non-REM sleep, Yukioka reported that the intrusion of the δ wave by the α wave could serve as an indicator of fibromyalgia symptoms, although this intrusion phenomenon was also detected in chronic fatigue syndrome and other diseases, and thus was not considered a pathological cause specific to fibromyalgia. The α wave is assumed to cause pain increase after waking up. Moldofsky *et al*. demonstrated that non-REM sleep disturbances cause fibromyalgia-like symptoms such as pain and fatigue in young healthy persons.

Neuropeptide abnormalities in fibromyalgia include serotonin abnormality. Moldofsky *et al*. reported that the blood tryptophan level was reduced in fibromyalgia. Russell *et al*. reported that peripheral blood and spinal fluid serotonin levels are lower in fibromyalgia patients and the level of the serotonin metabolite 5-hydroxyindoleacetic acid (5-HIAA) is reduced in the spinal fluid. Tryptophan is a precursor of serotonin. Serotonin is a brain neurotransmitter and functions in various types of neurons such as in raphe nuclei involved in pain suppression. Serotonin shortage generally causes various disorders. Focus is on the correlation of serotonin with the development of sleep disturbances and pain in fibromyalgia.

Another neuropeptide abnormality detected in fibromyalgia in addition to the serotonin abnormality is substance P abnormality. The finding that a substance called "substance P" was increased in the spinal fluid of fibromyalgia patients was reported in the meeting of the American College of Rheumatology in 2000. Substance P is also referred to as the "P substance". Substance P is a neuropeptide consisting of 11 amino acids, is a neurotransmitter in sensory nerves and is best known as a pain transmitter. This substance was identified from intestinal tract extracts and brain extracts, and is considered to be involved in various pathological conditions such as unilateral headache, pain, inflammation, vomiting, and anxiety.

There are reports on fibromyalgia-related functional abnormalities in terms of brain activity. A number of laboratories demonstrated by SPECT (Single Photon Emission Computed Tomography) that the blood flow was reduced in the thalamus and caudate nucleus of fibromyalgia patient brain (Non-Patent Document 3). Since the thalamus plays an important role in pain signal integration and the caudate nucleus is a tissue containing nociception-specific neurons, the reduced blood flow described above has drawn attention in the study of fibromyalgia pathogenesis. In addition, some reports in the United States describe that the blood flow was increased in the limbic system. As such, various abnormalities in the central nervous system such as sleep disturbances, neuropeptide abnormalities, and functional abnormalities in brain activity are observed in fibromyalgia, and the correlation of these abnormalities with fibromyalgia onset is being studied.

There are early reports on the genetic aspects of fibromyalgia, in particular, familial inheritance of fibromyalgia. A report describes that fibromyalgia was found in 71% of women and 35% of men among first-degree relatives (Non-Patent Document 3). There is a possibility that some genetic factors responsible for pain sensitivity, neurotransmission, and others dominant in women may be influencing the tendency to develop fibromyalgia.

The present inventor's group recently conducted genomic analysis of 200 patients, and identified a case where fibromyalgia had developed in both identical twins (Non-Patent Document 4). In addition, many other studies suggest that some genetic factors have an impact on fibromyalgia onset.

In some cases, fibromyalgia is thought to develop due to the combination of genetic factors and psychological and social factors. Many of the patients are in a state of depression. Stress-generating factors include physical exhaustion caused by irregular living habits, overwork, fatigue accumulation, and the like, and also psychological conflicts, frustrations, inadequate conditions, and frustrations due to unfair evaluations. One view is that fibromyalgia develops via a process triggered by the above-described physical and mental exhaustions together with physically traumatic experiences or such that make one feel muscle pains (Non-Patent Document 5). Events that adversely effect fibromyalgia were listed in the meeting of the American College of Rheumatology in 2000. Reports published in Japan also describe that, of 23 males and 116 females diagnosed with fibromyalgia, 12 males and 57 females, a total of 69 patients (49.6%) had a history of injury or surgery (Non-Patent Documents 4 and 6).

Furthermore, a comparison of fibromyalgia and chronic fatigue syndrome suggested that some lifestyle habits are involved in fibromyalgia onset (Non-Patent Document 7). According to the comparison, the risk is approximately halved by seven to eight hours of sleep as compared to nine or more hours of sleep. With only one to two hours of weekly exercise, the risk is about nine times greater as compared to active exercise. People who drink coffee every day have 3.7 times greater risk than those who do not drink coffee. 180 ml of alcohol a day reduces the risk to about one third compared to those who do not drink alcohol. Conversely, drinking too much alcohol increases the risk.

Meanwhile, some fibromyalgia patients have coexisting enthesitis. Enthesitis is a disorder characterized by inflammation in tendons and ligaments attached to bones, and is commonly seen in ankylosing spondylitis and reactive arthritis. Enthesitis is a disease accompanying pain, with symptoms likely to appear in the heel and plantar insertions of the Achilles tendon, and others. There is a strong suggestion that fibromyalgia may be triggered by enthesitis. Specifically, local pain in enthesitis can trigger the onset of fibromyalgia with systemic pain.

Basically, the administration of antidepressants is the first choice in fibromyalgia treatment. Currently, however, there is no definitive therapeutic method. Various approaches such as cognitive therapy and therapeutic exercise are considered necessary depending on symptoms and the degree of physical functional disorder. Symptoms of fibromyalgia pain and indefinite complaints are hardly alleviated through medications (such as ordinary analgesic agents and antirheumatic agents) alone. Psychological therapy such as lifestyle guidance, autogenic training, and cognitive-behavioral therapy are often effective when used in combination with antidepressants, anxiolytic agents, and muscle relaxants. Recently, there is a move to apply preexisting pharmaceutical agents to fibromyalgia (Patent Documents 1 and 2). In general however, even for pharmaceuticals with proven effects, there are patients who respond and who do not. Thus, there is an urgent need to increase options of fibromyalgia therapeutics.

Salazosulfapyridine is a clinically used antirheumatic drug. As a pharmacological action additional to its antirheumatic activity, salazosulfapyridine has also been reported to have an effect on the immune system. In this report, salazosulfapyridine suppressed the immune response to a T cell-dependent antigen in a dose-dependent manner, but hardly suppressed the immune response to a T cell-independent antigen (Non-Patent Document 8). Furthermore, salazosulfapyridine was also reported to suppress the production of IL-1 and IL-6 in peripheral blood-adhesive cells from chronic rheumatoid arthritis patients, and also IL-2 production in T cells in a dose-dependent manner (Non-Patent Documents 9 and 10). The mechanism of action for the antirheumatic activity of salazosulfapyridine has been interpreted as follows. Salazosulfapyridine acts on T cells and macrophages, and suppresses aberrant antibody production in rheumatoid arthritis patients by suppressing the production of cytokines (IL-1, 2, and 6) in the cells. Furthermore, salazosulfapyridine suppresses the activation of synovial cells, infiltration of inflammatory cells, and such, as well as the generation of reactive oxygen in polymorphonuclear leukocytes. Salazosulfapyridine is considered to exhibit antirheumatic activity by suppressing overall inflammation in the joints of chronic rheumatoid arthritis patients by these actions (Non-Patent Document 11).

As described above, salazosulfapyridine is effective for rheumatoid arthritis, and is also used as an agent for treating ulcerative colitis, regional enteritis, and nonspecific colitis. However, the effect of salazosulfapyridine on fibromyalgia was unknown.
Patent Document 1: WO2004/039383 (PCT/JP2003/013999)
Patent Document 2: Japanese Patent Application Kokai Publication No. (JP-A) 2006-76945 (unexamined, published Japanese patent application)
Non-Patent Document 1: Wolfe F, Smythe HA, Yunus MB, et al. The American College of Rheumatology 1990 Criteria for the Classification of Fibromyalgia. Arthritis Rheum 33(2):160-172, 1990
Non-Patent Document 2: Masao Yukioka. Fibromyalgia (2002) Sono Shindan to Chiryo (Its Diagnosis and Treatment) Ryumachi-byo Seminar XIV (Rheumatic Disease Seminar XIV), (Kanji Shichikawa, editorial supervisor), p. 49-58, Osaka, Nagai Shoten, 2003
Non-Patent Document 3: Masahiko Nishikai. Fibromyalgia syndrome. Ryumachika 27(3):298-304, 2002
Non-Patent Document 4: Wholeness Institute. Sen-ikintsusho to Tatakau (Fight against fibromyalgia) (Kusuki Nishioka, editorial supervisor), p. 117-124, Ishiyaku Pub, Inc.
Non-Patent Document 5: Masato Murakami and Kazuhiko Munakata. Pain Clinic 18(2):211-216, 1997
Non-Patent Document 6: Fusazo Urano. Ichijisei Sen-ikintsusho no Hotaichi no Ijyo (Abnormal levels of complements in Primary Fibromyalgia Syndrome), Abstract of the 42nd Annual General Assembly and Scientific Meeting of Japan College of Rheumatology, Rheumatism 38:304, 1998
Non-Patent Document 7: Yoshifuji Matsumoto. Fibromyalgia Syndrome. Nihon Rinsho 57(2):364-369, 1999
Non-Patent Document 8: Fujiwara M. et al. Immunopharmacol. 19:15, 1990
Non-Patent Document 9: Fujiwara M. et al. Japan. J. Pharmacol. 54:121, 1990
Non-Patent Document 10: Junko Hashimoto et al. Ensho (Inflammation) 11 (3):279, 1991
Non-Patent Document 11: Azulfidine EN Tablet 250 mg, Document attached to Azulfidine EN Tablet, p. 3, Revised June, 2005 (Eighth edition)

### Disclosure of the Invention

### [Problems to be Solved by the Invention]

The present invention was achieved in view of the circumstances described above. An objective of the present invention is to provide novel agents for treating fibromyalgia.

### [Means for Solving the Problems]

The present inventor conducted dedicated studies to achieve the objective described above. As described above, however, agents for treating fibromyalgia are very difficult to develop under the current circumstances, because both the pathogenetic cause and the onset mechanism of fibromyalgia remain to be clarified. Salazosulfapyridine is a pharmaceutical agent that is typically used as an antirheumatic drug. The present inventor administered salazosulfapyridine to fibromyalgia patients, and unexpectedly discovered that: (1) salazosulfapyridine is effective in fibromyalgia patients in whom neither the rheumatoid factor nor an immune disorder is detectable, in particular to relieve pain of enthesitis, and to relieve fibromyalgia pain in fibromyalgia accompanied by enthesitis; and (2) the combined use of salazosulfapyridine with a corticosteroid or a nonsteroidal anti-inflammatory agent is effective in patients in whom salazosulfapyridine alone is not effective. Accordingly, the present invention was completed. Specifically, the present invention relates to salazosulfapyridine-containing pharmaceutical agents for treating fibromyalgia and salazosulfapyridine-containing pharmaceutical agents for treating fibromyalgia accompanied by enthesitis. More specifically, the present invention provides:
[1] a pharmaceutical agent for treating fibromyalgia, which comprises salazosulfapyridine;
[2] the pharmaceutical agent of [1], wherein the fibromyalgia is accompanied by enthesitis;
[3] a pharmaceutical agent for treating enthesitis accompanying fibromyalgia, which comprises salazosulfapyridine;
[4] a pharmaceutical agent for treating pain accompanying fibromyalgia, which comprises salazosulfapyridine;
[5] the pharmaceutical agent of any one of [1] to [4], which is in an oral dosage form;
[6] the pharmaceutical agent of [5], which is a tablet;
[7] the pharmaceutical agent of [6], which is an enteric coated tablet;
[8] a pharmaceutical agent for treating fibromyalgia, which comprises in combination salazosulfapyridine and either a corticosteroid or a synthetic corticosteroid;
[9] the pharmaceutical agent of [8], which is provided as a kit comprising both of a pharmaceutical agent that comprises salazosulfapyridine and a pharmaceutical agent that comprises a corticosteroid or a synthetic corticosteroid;
[10] the pharmaceutical agent of [8] or [9], wherein the corticosteroid or synthetic corticosteroid is prednisolone, prednisolone ester, or prednisolone analog, or a salt thereof;
[11] a pharmaceutical agent for treating fibromyalgia, which comprises in combination salazosulfapyridine and a nonsteroidal anti-inflammatory agent;
[12] the pharmaceutical agent of [11], which is provided as a kit comprising both of a pharmaceutical agent that comprises salazosulfapyridine and a pharmaceutical agent that comprises a nonsteroidal anti-inflammatory agent;
[13] the pharmaceutical agent of [11] or [12], wherein the nonsteroidal anti-inflammatory agent is meloxicam;
[14] the pharmaceutical agent of [8] or [11], which is a combination drug;
[15] the pharmaceutical agent of any one of [8] to [14], wherein the fibromyalgia is accompanied by enthesitis;
[16] a method for treating fibromyalgia, which comprises the step of administering salazosulfapyridine;
[17] a method for treating fibromyalgia, which comprises the step of administering salazosulfapyridine and either a corticosteroid or a synthetic corticosteroid;
[18] a method for treating fibromyalgia, which comprises the step of administering salazosulfapyridine and a nonsteroidal anti-inflammatory agent;
[19] the therapeutic method of any one of [16] to [18], wherein the fibromyalgia is accompanied by enthesitis;
[20] a method for treating enthesitis accompanying fibromyalgia, which comprises the step of administering salazosulfapyridine;
[21] a method for treating enthesitis accompanying fibromyalgia, which comprises the step of administering salazosulfapyridine and either a corticosteroid or a synthetic corticosteroid;
[22] a method for treating enthesitis accompanying fibromyalgia, which comprises the step of administering salazosulfapyridine and a nonsteroidal anti-inflammatory agent;
[23] use of salazosulfapyridine in producing a pharmaceutical agent for treating fibromyalgia;
[24] use of salazosulfapyridine in producing a pharmaceutical agent for treating fibromyalgia accompanied by enthesitis; and
[25] use of salazosulfapyridine in producing a pharmaceutical agent for treating enthesitis accompanying fibromyalgia.

### Best Mode for Carrying Out the Invention

The present invention provides salazosulfapyridine-containing agents for treating fibromyalgia (hereinafter, "salazosulfapyridine-containing agents for treating fibromyalgia" are collectively called "pharmaceutical agents of the present invention"). Salazosulfapyridine acts effectively on joint inflammation accompanying autoimmune disorders (rheumatism) through the action mechanism described above. Thus, salazosulfapyridine is used as an antirheumatic drug. The present inventor demonstrated for the first time that salazosulfapyridine was effective for relieving pain due to enthesitis in fibromyalgia patients in whom neither the rheumatoid factor nor an immune disorder is detectable, as well as for relieving fibromyalgia pain in fibromyalgia accompanied by enthesitis.

Salazosulfapyridine is also referred to as sulfasalazine (INN, USAN). The chemical name according to IUPAC nomenclature is: 2-Hydoroxy-5-[4-(pyridine-2-ylsulfamoyl)phenylazo] benzoic acid. Salazosulfapyridine is a compound represented by the formula C₁₈H₁₄N₄O₅S, with a molecular weight of 398.39. The CAS registry number is 599-79-1. Salazosulfapyridine is listed in the Japanese Pharmacopoeia Fifteenth Edition, and thus its properties, identification and quantitation methods, and other information is available in the Japanese Pharmacopoeia.

Salazosulfapyridine was synthesized by a collaboration between Dr. N. Svartz and Pharmacia (currently, Pfizer). In Japan, salazosulfapyridine was developed in 1984 as an agent for treating rheumatoid arthritis, and was approved on 29th September, 1995. It is available on the market under the trade name of Azulfidine. Currently, in addition to Azulfidine, several domestic pharmaceutical companies are selling salazosulfapyridine preparations, which are available under the trade names of Eminapyrin, Safildine, Salazopyrin, Slama, Soaresin, and Lanofen. Salazosulfapyridine preparations have been approved for other indications in addition to rheumatoid arthritis. Of the salazosulfapyridine preparations listed above, Salazopyrin, Eminapyrin, Slama, and Lanofen are agents for treating ulcerative colitis, regional enteritis, and nonspecific colitis.

As described above, fibromyalgia is generally diagnosed based on the criteria established by the American College of Rheumatology. The fibromyalgia classification criteria set forth in 1990 by the American College of Rheumatology are as follows.
(1) Widespread pain has been present for three months or longer. Pain is considered widespread when all of the following are present: pain in the left side of the body, pain in the right side of the body, pain above the waist, pain below the waist, and axial skeletal pain (cervical spine, anterior spine, thoracic spine, or low back).
   In this definition, pain in the right and left shoulders and buttocks is included in pain in the right and left side of the body, respectively, and low back pain is considered lower body segment pain.
(2) On digital palpation with an approximate force of 4 kg, pain must be present in 11 or more of 18 tender points. Eighteen tender point sites are:
   *occiput*: bilateral, at the suboccipital muscle insertions;
   *low cervical*: bilateral, at the anterior aspects of the intertransverse spaces at C5-C7;
   *trapezius*: bilateral, above the scapula spine near the upper border;
   *second rib*: bilateral, at the second costochondral junctions, just lateral to the junctions on upper surfaces;
   *lateral epicondyle*: bilateral, 2 cm distal to the epicondyles;
   *gluteal*: bilateral, in upper outer quadrants of buttocks in anterior fold of muscle;
   *greater trochanter*: bilateral, posterior to the trochanteric prominence; and
   *knee*: bilateral, at the medial fat pad proximal to the joint line.
   When a patient feels pain on digital palpation, the tender point is considered "positive". Tender is not considered "positive" when there is no pain under normal conditions.

The presence of a second clinical disorder does not exclude the diagnosis of fibromyalgia.

When the two criteria described above are met, patients are diagnosed as having fibromyalgia. Clinically, however, patients may be diagnosed as having fibromyalgia by specialists even if they do not meet the two criteria. Like the clinical judgment, the pharmaceutical agents of the present invention are also applicable not only to patients who meet the above criteria but also to those diagnosed as having fibromyalgia by specialists.

The pharmaceutical agents of the present invention can relieve various symptoms of fibromyalgia. Of the various symptoms of fibromyalgia, the pharmaceutical agents of the present invention are particularly effective for relieving pain, and in particular effective for relieving pain in enthesitis. The pharmaceutical agents of the present invention can relieve pain in tendon insertions of any body part, as long as it is caused by enthesitis accompanying fibromyalgia. Furthermore, the agents are also effective for treating fibromyalgia pain in fibromyalgia accompanied by enthesitis.

As is evident from the fact that Salazosulfapyridine is already available in the market, salazosulfapyridine can be formulated by known techniques. The dosage form may be oral preparations (tablets, granules, liquids, capsules, and such), injections, patches, liniments, suppositories, creams, suspensions, emulsions, or ointments, as long as it allows the active ingredient to reach the affected areas. Oral preparations are preferred from the viewpoint of relatively low invasiveness towards patients. Oral preparations may be formulated as enteric coated drugs. Additives used for formulation can be selected from pharmaceutically acceptable materials depending on the purpose. The "pharmaceutically acceptable additives" include excipients, diluents, expanders, disintegrating agents, stabilizers, preservatives, buffers, emulsifiers, aromatics, colorants, sweeteners, viscosity increasing agents, flavors, solubilizing agents, coating agents, binders, and other additives.

The dose of a pharmaceutical agent of the present invention can be determined by considering the safety-efficacy balance, administration route, patient's age and weight, etc. When a pharmaceutical agent of the present invention is administered orally, the daily salazosulfapyridine dose is, for example, 0.01 to 10 g, preferably 0.1 to 5 g, more preferably 0.5 to 3 g, and still more preferably 1 to 2 g. When the agent is a parenteral preparation, the appropriate dose can be determined by considering the absorption, distribution, metabolism, and excretion depending on each preparation.

The present inventor demonstrated that when administered in combination with synthetic corticosteroids, salazosulfapyridine produced a significant therapeutic effect on fibromyalgia patients on whom the administration of salazosulfapyridine alone produced no therapeutic effect. Thus, the present invention also provides pharmaceutical agents for treating fibromyalgia, which comprise a combination of salazosulfapyridine and either a corticosteroid or synthetic corticosteroid.

Corticosteroid is in general a generic term for steroidal hormones that are produced or secreted by the adrenal cortex. Corticosteroids are roughly divided into glucocorticoids, mineralocorticoids, and sex hormones based on their activities. Representative natural corticosteroids are, for example, cortisone, cortisol, corticosterone, 11-deoxycorticosterone, and aldosterone. Many synthetic corticosteroids having a structure and activity similar to those of natural corticosteroids have been developed and put into practical use. Such synthetic corticosteroids include, for example, prednisolone, prednisone, methyl prednisolone, dexamethasone, betamethasone, paramethasone, and salts thereof. Corticosteroids and synthetic corticosteroids used in the present invention are not particularly limited as long as they can enhance the therapeutic effect in the treatment of fibromyalgia when used in combination with salazosulfapyridine. Corticosteroids and synthetic corticosteroids having glucocorticoid activity are preferred, including, for example, prednisolone, prednisolone esters, prednisolone analogs, and salts thereof. However, the corticosteroids and synthetic corticosteroids are not limited to these examples. Any corticosteroid or synthetic corticosteroid can be used in the present invention, as long as it has the activity of enhancing the therapeutic effect described above. Known prednisolone esters and analogs, and salts thereof include, for example, methylprednisolone, methylprednisolone succinate, prednisolone acetate, prednisolone succinate, prednisolone sodium succinate, prednisolone sodium phosphate, prednisolone valerate acetate, and prednisolone farnesylate.

When salazosulfapyridine is used in combination with corticosteroids or synthetic corticosteroids in the present invention, they may be administered as separate preparations or as a single combination drug. For convenience of use, a salazosulfapyridine preparation may be combined with a corticosteroid or a synthetic corticosteroid preparation, and made into a therapeutic kit for fibromyalgia with instructions for use, etc.

When a corticosteroid or a synthetic corticosteroid is formulated, its administration route, dosage form, and dose can be appropriately determined by considering the composition, patient's symptoms, age, weight, and such. When the ingredient is already clinically used, it is basically possible to refer to the clinical administration route, dosage form, and dose.

Furthermore, the present inventor demonstrated that therapy using in combination salazosulfapyridine and a nonsteroidal anti-inflammatory agent is effective for fibromyalgia patients on whom the administration of salazosulfapyridine alone produces no therapeutic effect. Thus, the present invention also provides pharmaceutical agents for treating fibromyalgia, which comprise in combination salazosulfapyridine and a nonsteroidal anti-inflammatory agent.

Nonsteroidal anti-inflammatory drug (NSAID) is a generic term for anti-inflammatory agents other than steroids. Nonsteroidal anti-inflammatory drugs exercise anti-inflammatory functions by inhibiting prostaglandin synthesis via cyclooxygenase (COX) inhibition. Conventional nonsteroidal anti-inflammatory drugs inhibit both COX-1 and COX-2, and thus tend to induce adverse effects such as gastrointestinal disturbances. It is assumed that the adverse effects such as gastrointestinal disturbances of nonsteroidal anti-inflammatory drugs can be reduced by selectively inhibiting COX-2. Based on this, COX-2-selective nonsteroidal anti-inflammatory drugs have been developed. COX-2-selective nonsteroidal anti-inflammatory drugs such as etodolac and meloxicam have already been clinically used in Japan. In other countries, COX-2-selective nonsteroidal anti-inflammatory drugs such as celecoxib, rofecoxib, valdecoxib, parecoxib, etoricoxib, and lumiracoxib are under development or already in use.

Known nonsteroidal anti-inflammatory agents available in the market include, for example, sodium salicylate, acetylsalicylate, salicylamide, flufenamic acid aluminum, mefenamic acid, tolfenamic acid, diclofenac sodium, sulindac, amfenac sodium, indomethacin, indomethacin farnesyl, proglumetacin maleate, acemetacin, nabumetone, etodolac, mofezolac, ibuprofen, ketoprofen, flurbiprofen, flurbiprofen axetil, oxaprozin, fenoprofen calcium, tiaprofenic acid, naproxen, pranoprofen, loxoprofen sodium, alminoprofen, zaltoprofen, bucolome, piroxicam, ampiroxicam, tenoxicam, meloxicam, lomoxicam, epirizole, tiaramide hydrochloride, emorfazone, and extracts from cutaneous tissue of rabbit inoculated with vaccinia virus. Nonsteroidal anti-inflammatory agents that can be used in the present invention are not particularly limited as long as they increase the therapeutic effect on fibromyalgia when used in combination with salazosulfapyridine. Such nonsteroidal anti-inflammatory agents are not limited to the above examples, and it is possible to use any nonsteroidal anti-inflammatory agent.

When salazosulfapyridine is used in combination with nonsteroidal anti-inflammatory agents in the present invention, they may be administered as separate preparations or as a single combination drug. For convenience of use, a salazosulfapyridine preparation may be combined with a preparation of a nonsteroidal anti-inflammatory agent, and made into a therapeutic kit for fibromyalgia with instructions for use, and such.

When a nonsteroidal anti-inflammatory agent is formulated, its administration route, dosage form, and dose can be appropriately determined by considering the composition, patient's symptoms, age, weight, and such. When the ingredient is already clinically used, it is basically possible to refer to the clinical administration route, dosage form, and dose.

All prior art references cited in this specification are incorporated herein by reference.

### Examples

Hereinbelow, the present invention is specifically described in the context of Examples; however, it is not to be construed as being limited thereto.

### [Example 1] Treatment of fibromyalgia by administering salazosulfapyridine alone

Azulfidine^{™} EN tablets, a commercially available salazosulfapyridine preparation, were administered to patients diagnosed with fibromyalgia. The follow-up examination revealed that the salazosulfapyridine administration produced a significant therapeutic effect.

Azulfidine was administered to ten fibromyalgia patients at 500 to 1,000 mg/day for two to ten weeks. This significantly alleviated the subjective symptoms. Tender points were decreased to 75.5%, and the percent alleviation of VAS was 60.7%.

### [Example 2] Treatment of fibromyalgia using in combination salazosulfapyridine and a corticosteroid or a nonsteroidal anti-inflammatory agent

### (Case 1)

Treatment with oral Luvox, Lexotan, and Cercine was commenced due to panic attacks. Back pain appeared after two years, and even walking became difficult due to systemic pain. Blood tests suggested neither inflammation nor connective tissue disease. A complete full-body check up revealed no abnormalities.

However, there was pressure pain in the occiput, trapezius, supraspinatus, gluteal region, etc. Thus, fibromyalgia was suspected and treatment was started with the following prescription:

### Prescription:

Amlodin (5 mg), one tablet
Voltaren (25 mg), three tablets
Depas (0.5 mg), one tablet
Zantac (150 mg), two tablets
Luvox (25 mg), three tablets
Lexotan (2 mg), three tablets
Cercine (2 mg), three tablets

Although the patient was treated with the above prescription, 14/18 tender points were positive and there was pressure pain in tendon insertions and difficulty in falling asleep. Thus, the following was additionally prescribed. RA factor was negative, and there was neither inflammation nor swelling.

### Additional prescription:

Azulfidine (500 mg), two tablets
Predonine (generic name: prednisolone) (5 mg), one tablet

Two weeks after the start of the additional prescription, tender points decreased from 14/18 to 3/18. Azulfidine thus produced a significant pain-relieving effect, and was effective for pressure pain in tendon insertions in particular.

### (Case 2)

An early symptom was a complaint of fatigue, which gradually became worse. After seven months, the patient had difficulty going outside. During this period, the patient was subjected to thorough clinical examinations such as blood tests, and examinations in a specialized medical center for connective tissue disease. The test results were all normal. However, symptoms exacerbated during the outpatient period. Severe pain in the whole body, slight fever, hives, malaise, and fatigue appeared, and the patient had difficulty walking.

Symptoms were slightly relieved by oral treatment with only vitamin C, Mobic (generic name: meloxicam), and stomach medicine, resting at home, and outpatient care. However, the patient still had difficulty going outside alone, and thus visited the hospital of the present inventor.

On the first visit, a drip infusion of one ampule of Neurotropin was prescribed. After two weeks, pain was relieved due to Mobic (analgesic agent). However, since VAS (pain scale) was 5.5/10, the prescription was changed as follows.

### Prescription change:

Neurotropin, three tablets
Azulfidine (500 mg), one tablet

Ten weeks after the first visit, pain was significantly relieved and VAS (pain scale) became 1/10 to 2/10. Thereafter, Azulfidine treatment was continued. Eight months after the first visit, tender points decreased from 18/18 to 4/18. Pain was almost completely alleviated, and the patient was able to go out.

### (Case 3)

The patient sprained a leg in December, 2005. Thereafter, complaining of pain in the right elbow and a tingling sensation in the arm below the elbow, the patient visited the orthopedics. Cervical X-ray was normal. Pain and morning stiffness appeared in the toes in May of the following year. The patient then visited a neurological clinic of a university hospital. Rheumatoid factor was negative, and all other complete clinical tests were normal. Since there was pressure pain at fibromyalgia tender points, the patient was suspected to have fibromyalgia. Thus, the patient visited the hospital of the present inventor.

### Prescription at the start of treatment (21 st July, 2006):

Mobic (10 mg), one tablet/day (single administration/day)
Neurotropin, four tablets/day (the daily dose was divided and given twice a day).

The following additional prescription was started one week after the start of treatment (27th July, 2006):
Azulfidine (250 mg), two tablets/day (the daily dose was divided and given twice a day).

The following additional prescription was commenced two weeks after the start of treatment (10th August, 2006):
Predonine (5 mg), one tablet/day (single administration/day); additional prescription.

Pain tended to alleviate about five weeks after the start of treatment (24th August, 2006).

The patient had been taking leave from work, but was planning to return to work because the symptoms were alleviated.

### (Case 4)

A 38-year-old woman complained of the following clinical signs: stiff neck, early morning awakening, malaise, fatigue, systemic joint pain upon awakening, stiffness, pain and numbness in both elbows down to the fingers, and pain in the back of both knees down to the toes. Azulfidine and other drugs were given. In this case, the pain scale VAS was alleviated from 10 to 2 in about two months.

The history before visiting the hospital of the inventor is as follows.

The patient fell down and sprained her left ankle in December, 2005. Pain appeared in the right elbow on the same night. Cervical X-ray was normal.

After one week, pain spread from the elbow to the wrist and fingers.

After two weeks, pain spread to the left elbow, wrist, and fingers as well. The patient visited the orthopedics, but cervical X-ray and blood tests for rheumatism were all normal.

In January, 2006, the patient was subjected to medical tests. The results were normal.

In February, 2006, the patient developed depression, and was thus hospitalized from February to April.

In May, 2006, the patient visited the neurological clinic due to pain and morning stiffness of toes (with FMS tender points).

In July, 2006, the patient visited the hospital of the present inventor for the first time.

The treatment history is as follows.

On 21st June, 2007, there were 14 tender points among 18; pain was present in tendon insertions. VAS was 10. One tablet of Mobic (10 mg) and four tablets of Neurotropin (four Neurotropin units) were prescribed.

On 27th July, 2007, VAS was 10. Two tablets of Azulfidine (250 mg) were additionally prescribed.

On 10th August, 2007, the patient's mood was stable; VAS was 9. One tablet of Predonine (5 mg) was additionally prescribed.

On 24th August, 2007, the patient gave the impression that her pain had been relieved (VAS 9). The same medicine was prescribed.

On 21 st September, 2007, there were 3 tender points among 18; VAS was 2. The same medicine was prescribed.

On 19th October, 2007, VAS was 2. The same medicine was prescribed.

On 16th November, 2007, VAS was 2. Catlep was additionally prescribed.

### Industrial Applicability

The present invention provides novel agents for treating fibromyalgia. The present invention can provide effective methods for treating fibromyalgia, which was believed to have no effective drugs.

## Claims

1. A pharmaceutical agent for treating fibromyalgia, which comprises salazosulfapyridine.

2. The pharmaceutical agent of claim 1, wherein the fibromyalgia is accompanied by enthesitis.

3. A pharmaceutical agent for treating enthesitis accompanying fibromyalgia, which comprises salazosulfapyridine.

4. A pharmaceutical agent for treating pain accompanying fibromyalgia, which comprises salazosulfapyridine.

5. The pharmaceutical agent of any one of claims 1 to 4, which is in an oral dosage form.

6. The pharmaceutical agent of claim 5, which is a tablet.

7. The pharmaceutical agent of claim 6, which is an enteric coated tablet.

8. A pharmaceutical agent for treating fibromyalgia, which comprises in combination salazosulfapyridine and either a corticosteroid or a synthetic corticosteroid.

9. The pharmaceutical agent of claim 8, which is provided as a kit comprising both of a pharmaceutical agent that comprises salazosulfapyridine and a pharmaceutical agent that comprises a corticosteroid or a synthetic corticosteroid.

10. The pharmaceutical agent of claim 8 or 9, wherein the corticosteroid or synthetic corticosteroid is prednisolone, prednisolone ester, or prednisolone analog, or a salt thereof.

11. A pharmaceutical agent for treating fibromyalgia, which comprises in combination salazosulfapyridine and a nonsteroidal anti-inflammatory agent.

12. The pharmaceutical agent of claim 11, which is provided as a kit comprising both of a pharmaceutical agent that comprises salazosulfapyridine and a pharmaceutical agent that comprises a nonsteroidal anti-inflammatory agent.

13. The pharmaceutical agent of claim 11 or 12, wherein the nonsteroidal anti-inflammatory agent is meloxicam.

14. The pharmaceutical agent of claim 8 or 11, which is a combination drug.

15. The pharmaceutical agent of any one of claims 8 to 14, wherein the fibromyalgia is accompanied by enthesitis.

16. A method for treating fibromyalgia, which comprises the step of administering salazosulfapyridine.

17. A method for treating fibromyalgia, which comprises the step of administering salazosulfapyridine and either a corticosteroid or a synthetic corticosteroid.

18. A method for treating fibromyalgia, which comprises the step of administering salazosulfapyridine and a nonsteroidal anti-inflammatory agent.

19. The therapeutic method of any one of claims 16 to 18, wherein the fibromyalgia is accompanied by enthesitis.

20. A method for treating enthesitis accompanying fibromyalgia, which comprises the step of administering salazosulfapyridine.

21. A method for treating enthesitis accompanying fibromyalgia, which comprises the step of administering salazosulfapyridine and either a corticosteroid or a synthetic corticosteroid.

22. A method for treating enthesitis accompanying fibromyalgia, which comprises the step of administering salazosulfapyridine and a nonsteroidal anti-inflammatory agent.

23. Use of salazosulfapyridine in producing a pharmaceutical agent for treating fibromyalgia.

24. Use of salazosulfapyridine in producing a pharmaceutical agent for treating fibromyalgia accompanied by enthesitis.

25. Use of salazosulfapyridine in producing a pharmaceutical agent for treating enthesitis accompanying fibromyalgia.
